# EUROPEAN PATENT SPECIFICATION

(11) **EP 1 263 358 B1**
(45) Date of publication and mention of the grant of the patent: **10.06.2009**
(21) Application number: 01922243.9
(22) Date of filing: 14.03.2001
(51) Int. Cl.: A61F 2/78

(54) **COMPOSITE ELASTIC MATERIAL**
ELASTISCHES VERBUNDMATERIAL
MATIERE ELASTIQUE COMPOSITE

(30) Priority: 14.03.2000 US 189039 P
(43) Date of publication of application: 11.12.2002
(73) Proprietor: Ossur HF, 110 Reykjavik (IS); Ossur, Inc., Aliso Viejo, CA 92656 (US)
(72) Inventor: JANUSSON, Hilmar, IS-170 Seltjarnarnesi (IS); THORSTEINSSON, Freygarour, 110 Reykjavik (IS); ASGEIRSSON, Sigurour, A., 210 Gardabaer (IS); EINARSSON, Palmi, 270 Mosfellsbaer (IS)
(74) Representative: Klunker, Hans-Friedrich
(86) International application number: PCT/US2001/005805
(87) International publication number: WO 2001/067984

(56) References cited:
- EP-A1- 0 101 779
- WO-A-88/00032
- WO-A-98/49977
- DE-A1- 19 823 753
- US-A- 3 634 136
- US-A- 4 923 474
- US-A- 5 507 834
- US-A- 5 571 208
- US-A- 5 830 237
- US-A1- 4 463 118
- US-A1- 5 603 122
- US-A1- 5 738 812
- US-B1- 6 183 766

## Description

### 1. Field of the Invention

This invention relates to composite elastic materials useful for prosthetic applications, and prosthetic devices made therefrom.

### 2. Background of the Invention

Various silicone elastomer materials have been proposed for use in prosthetic devices such as suction liners of the type described in U.S. Patent No. 4,923,474 granted to Klasson and Kristinsson on May 8, 1990. Other examples of such suction liners include U.S. Patent No. 5,728,168 granted March 17, 1998 to Laghi et al. and U.S. Patent No. 5,830,237 granted to Kania November 3, 1998.

A silicone elastomer suspension liner for a prosthesis is described in WO 98/49977. It includes a soft inner silicone elastomer layer and a relatively harder outer silicone elastomer layer. A elasticity controlling matrix is provided within the silicone elastomer between the layers. The layers may be cured and add up to an air-tight liner. To place the liner over the distal end of a residual limb, it may be rolled up.

Composite elastomer materials useful for both suction liners and sealing sleeves are disclosed in U.S. Patent No. 5,571,208 granted November 5, 1996 to Caspers.

It is highly desirable to provide a relatively soft cushion in contact with or adjacent the skin of the user of a prosthetic device for comfort. However, the cushion must be relatively inert with respect to the skin of the user, be readily washable and feel comfortable. Silicone elastomers and silicone gels are generally known for such applications and function satisfactorily as suction liners in sleeves both when the silicone material is used alone and when it is used in combination with an outer stretchable fabric covering.

It has been observed, however, that as the thickness of the silicone elastomer cushion grows, so does the weight of the suction liner. It is highly desirable to obtain the soft cushioning effect of a silicone elastomer in a suction liner application while reducing the overall weight of the liner attributable to the silicone elastomer.

In suction sleeves formed with a silicone elastomer liner, it is desirable to form a relatively thin posterior wall and a relatively thick anterior wall to provide cushioning on the anterior wall while avoiding interference with movement of the prosthetic user, particularly with a below-the-knee amputee. Such a configuration, on the other hand, generally requires a discontinuity of curvature in the transition regions between the anterior and posterior walls. This rapid transition in wall thickness and curvature can be a source of discomfort to a prosthetic user and it is desirable to avoid such rapid change in elastomer wall thickness in these transition regions.

### BRIEF SUMMARY OF THE INVENTION

The present invention is a composite elastic material particularly useful for prosthetic applications and that is formed of at least one cured silicone elastomer layer containing silicone oil and hollow microspheres dispersed throughout the elastomer layer.

The composite elastic material is in the form of a tubular prosthetic suction liner having a closed distal end and dimensioned and configured so as to be rollable onto a distal end of a residual limb of a prosthetic device user, with a silicone elastomer layer on the interior of the liner and the elasticized fabric on the exterior of the liner. Such residual limb receiving liners are used between a socket of a prosthetic appliance and the residual limb of the appliance user.

The composite elastic material preferably also includes an elasticized fabric layer intimately bonded to one side of the silicone elastomer layer described above.

The composite elastic material also may be a sealing sleeve that is tubular in form and open at opposed ends, with the silicone elastomer layer covering the inside wall of the sleeve and the elasticized fabric covering the exterior of the sleeve. Such a sleeve is dimensioned and configured to be installed as a suction seal between a prosthetic device and the residual limb of the prosthetic device user, with the silicone elastomer layer facing towards the location of the residual limb with the upper end of the prosthetic device located within the sleeve.

When used in a suction liner, the composite elastic material may include a distension controlling reinforcement matrix embedded in the silicone elastomer layer over a distal end area of the suction liner, wherein the reinforcement matrix, for example a circular knit textile, contains reinforcement elements that provide substantial stiffness against elongation of the liner in a direction along the liner length but which do not provide substantial resistance against distension of the silicone elastomer layer in directions transverse to the liner length.

A rigid prosthetic connector element may be attached to the distal end of the suction liner by embedding the connector element in a cured silicone elastomer distal end cap adhered to the distal end of the suction liner. The connector is sufficiently exposed while embedded in the end cap so as to provide access to a prosthetic connector pin that may be fastened to the connector. The silicone elastomer distal end cap may be formed of a silicone elastomer having a higher durometer than the composite elastic silicone elastomer layer to provide a more rigid support zone at the distal end of the suction liner.

The microspheres used in the composite elastic material preferably are expanded polymeric shells having a density of .005 g/cm³ to 1.25 g/cm³, preferably .05 g/cm³.

The silicone elastomer layer preferably comprises, by weight, 50 - 99.4% silicone elastomer; .5 - 45% silicone oil; and .1 - 5% microspheres. Preferably, the ratio of silicone elastomer, silicone oil and microspheres is: 77.25% silicone elastomer; 10% silicone oil and .75% microspheres (by weight).

Preferably, the silicone elastomer material of the composite elastic material is blended with one or more skin treating agents such as Vaseline and aloe vera. For example, the silicone elastomer may be blended with up to 3% aloe vera by weight of the silicone elastomer layer, with the balance of the skin treatment agent constituting Vaseline, so that the silicone elastomer layer is blended with skin treatment agents up to about 20% by weight of the silicone elastomer layer.

The composite elastic material containing silicone elastomer, silicone oil, and microspheres preferably has a density of 5 g/cm³ to 1.3 g/cm³; a tensile strength of at least .1 Pa; a durometer (00) of 13 - 62; a 100% modulus of 5 kPa to 250 kPa; and a compression set of 0 to 30.

The invention also contemplates a suction liner formed of the composite elastic material in accordance with the invention wherein the suction liner is tapered conically inwardly towards its distal end from its open proximal end, and wherein the sleeve has a circular outer wall having radii of curvature centered along a first longitudinal sleeve axis of external symmetry extending longitudinally centrally within the sleeve, a circular curved inside anterior wall portion extending along a sleeve length and having first radii of curvature centered on a second longitudinal axis of anterior curvature extending longitudinally along said sleeve length and a circular curved inside posterior wall portion having second radii of curvature centered on a third longitudinal axis of posterior curvature extending along said sleeve length; said first, second and third longitudinal axes lying in a common longitudinally and transversely extending plane bisecting the anterior and posterior wall portions, and wherein said second and third axes are spaced apart at predetermined offset distance on opposed sides of said first axis to thereby define an anterior wall portion that is thicker along the sleeve length than the posterior portion; and further wherein the anterior and posterior wall portions intersect each other along said sleeve length on the sleeve interior along diametrically opposed inner transition wall portions that extend tangentially relative to the adjoining anterior and posterior wall portions along said sleeve length, whereby the interior wall of the suction sleeve along the transition wall portions is free of rapid changes in thickness, curvature or cross-section profile.

Preferably, the second and third radii of curvature are equal to each other along their respective second and third axes.

A spherical curved inside distal wall portion may be provided within the suction liner, said distal wall portion joining the adjoining interior wall of the suction liner along a tangency that forms a smooth transition between the inside distal wall portion and the adjoining interior wall of the suction liner. The thickness of the adjacent interior wall of the suction liner may be the same as the thickness of the anterior wall of the suction liner.

When provided with an elasticized fabric layer bonded on one side of the silicone elastomer layer, a thin continuous coating of second cured elastomer material is provided on the elasticized fabric between the fabric and the principal silicone elastomer layer. The thin coating of the second elastomer material partially penetrates and is embedded in the fabric layer and forms a continuous coating over the textile material between the textile and the principal silicone elastomer layer. The silicone elastomer coating material is fully stretchable elastically at least to same extent as the elasticized fabric layer to which it is attached and adhered.

When the composite elastic material is formed into a sealing sleeve with an elasticized textile fabric layer bonded on one side of the silicone elastomer layer, the textile fabric may be a circular rib knit formed principally of Nylon with a small amount of Lycra or other stretchable fiber. A secondary coating of cured silicone elastomer material may be used between the principal silicone elastomer layer and the fabric, in the same manner as described above.

### BRIEF DESCRIPTION OF THE DRAWINGS

With reference to the appended drawings which illustrate preferred embodiments of the invention:
Figure 1 is a cross-section of a suction liner constructed in accordance with the present invention and utilizing a composite elastic material also made in accordance with the invention;
Figure 2 is a distal end view of the suction liner shown in Figure 1;
Figure 3 is a section taken along line III-III of Figure 1;
Figure 4 is an enlarged view of Figure 3;
Figure 5 illustrates a sealing liner made with a composite elastic material in accordance with the present invention;
Figure 6 is a side elevational view of another example of a suction liner made with a composite elastic material in accordance with the present invention;
Figure 7 is an isometric view of the suction liner shown in Figure 6;
Figure 8 is a front elevation view of the suction liner shown in Figure 6;
Figure 9 is a section view taken along line IX-IX of Figure 8: and
Figure 10 is a section view taken along line X-X of Figure 8.

### DETAILED DESCRIPTION OF PREFERRED EMBODIMENTS OF THE INVENTION

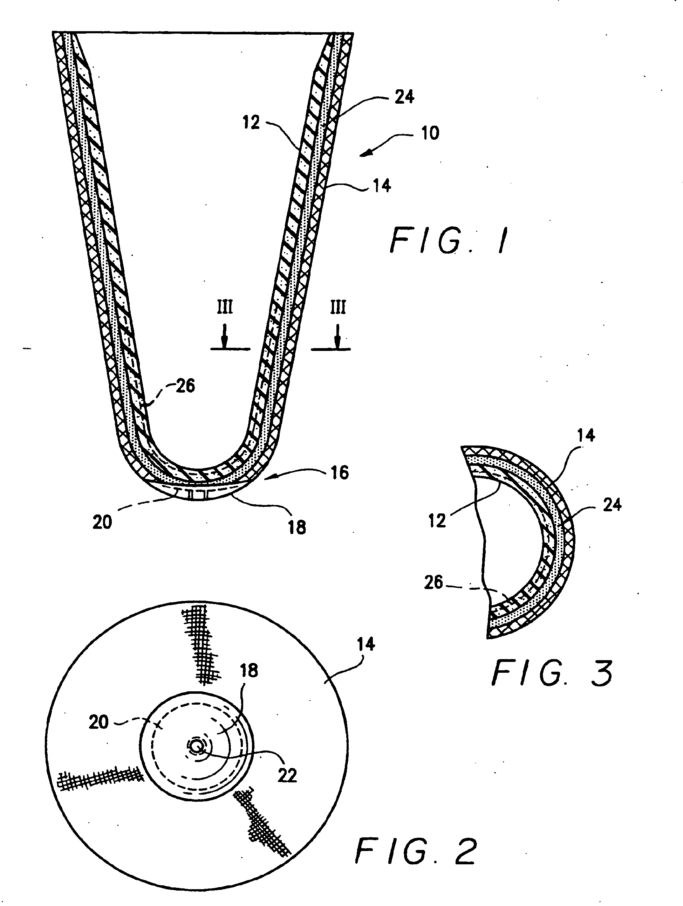

Figure 1 schematically illustrates in a cross-section view a prosthetic suction liner 10 formed in part of a composite elastic material 12 on its interior surface and an elasticized fabric layer 14 on its exterior surface at least up to its distal end area 16, where a distal end cap 18 having embedded therein a rigid prosthetic connector 20 formed, for example, of aluminum or other metal, or rigid plastic such as Nylon.

The liner 10 is formed as a close ended tapered tubular element, as is conventional for such suction liners. The distal end cap 18 firmly joins the prosthetic connector 20 to the suction liner 10 while providing a cushioning and stabilizing surface at the distal end of the liner. The prosthetic connector 20 includes preferably a threaded aperture 22 for providing access to a threaded prosthetic pin connector in a manner well known in the art.

Figure 2 shows the suction liner in an end view as seen from the distal end of the liner.

Figure 3 is a cross-section view taken along line III-III of Figure 1 and shows the composite elastic material 12, the elasticized fabric layer 14, and a second thin continuous coating of silicone elastomer material 24 partially embedded in the elasticized fabric layer 14 while not completely penetrating the fabric layer. The intermediate coating 24 is bonded on its opposite side to the composite elastic material 12, whereby the entire assembly of composite elastic material 12, elastomer coating 24 and elasticized fabric 14 is at least freely radially elastically distendable.

The composite elastic material layer 12 may have embedded therein a matrix of fibers 26 or other suitable stiffness reinforcement having properties such that the composite elastic layer 12 is rendered relatively stiff against longitudinal elongation while it is freely distendable radially of the suction liner for use in liner applications when axial elongation of the liner must be limited.

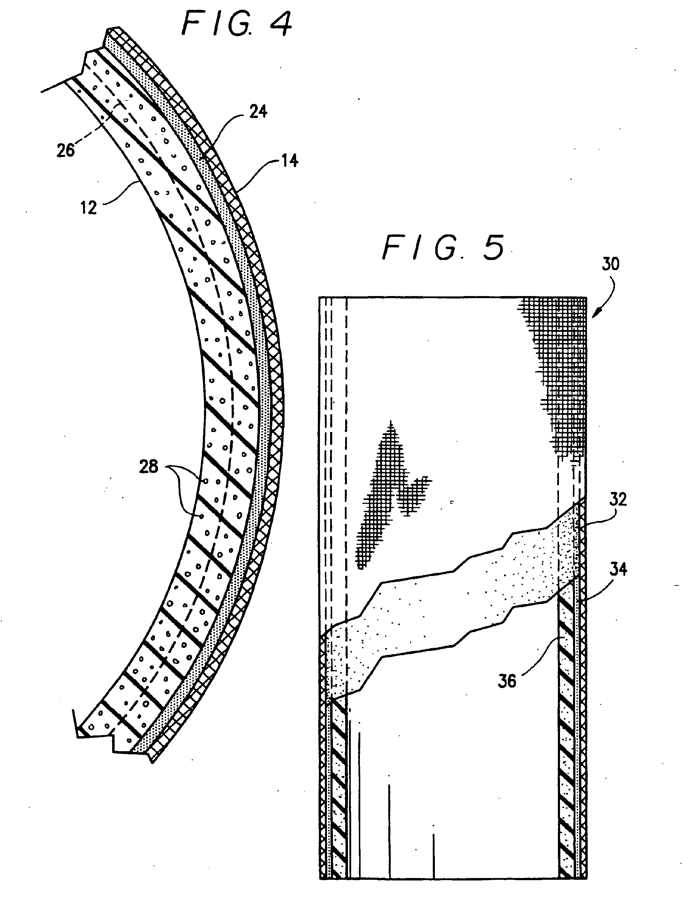

Figure 4 is an enlarged view of Figure 3 and shows the composite elastic material made in accordance with the present invention in more detail as well as the wall section of a suction liner 40 made with such material. Layer 12 is the composite elastic material comprising a cured silicone elastomer containing silicone oil and hollow microspheres 28 dispersed throughout the silicone elastomer layer.

The illustrations in Figures 3 and 4 also show the reinforcement fibers 26 embedded in the silicone elastomer layer, but it should be understood that such reinforcement fibers are optional and extend over a distal portion of the liner to limit axial distension of the liner in such distal portion. The reinforcement fibers 26, of course, do not constitute a portion of the basic composite elastic material described above.

More specifically, the composite elastic material layer 12 itself is regarded as a new and useful composite elastic material independently of the fibers 28, the outer fabric 14 and the intermediate coating.

In a preferred example, the composite elastic material 12 is formed principally of a silicone elastomer obtainable from NuSil Technology of Carpinteria, California under Product Designation CF13-2188. For a fuller description of the silicone material, reference may be made to U.S. Patent No. 6.136,039 granted October 24, 2000, owned by the assignee of the invention described herein.

Embedded within the silicone elastomer material of layer 12 are hollow thermoplastic microspheres consisting of a polymeric shell with an enclosed blowing agent. The specific thermoplastic microspheres utilized in this example of the invention are expanded microspheres obtained from AKZO NOBEL of Sweden under the trade name EXPANCEL®, Product No. 551DE.

The microspheres 28 preferably have a density of .005 g/cm³ to 1.25 g/cm³, preferably .05 g/cm³.

For a fuller understanding of the formulation of EXPANCEL® microspheres, reference may be made to EXPANCEL® Technical Bulletin 29 and the EXPANCEL® product specification and material safety data sheets, all available from AKZO NOBEL, S-850, 13. Sundsvall, Sweden.

The silicone oil included in the composite elastic material may be obtained from GE Bayer Silicones GmbH of DJ1 368 Leverkusen, Germany under Product Name Baysilone Fluid M350.

In a preferred form, the composite elastic layer 12 comprises 50 - 99.4% by weight of silicone elastomer, preferably 77.25%; .5 - 45% by weight of silicone oil, preferably 10%; and .1 - 5% by weight of microspheres, preferably .75%.

The composite elastic layer 12 also may include one or more skin treatment agents blended into the silicone elastomer, for example Vaseline and aloe vera. In a preferred example, up to 20% by weight of the composite elastic layer, preferably 11.9%, may be Vaseline and up to 3%, preferably 1%, may be a secondary skin treatment agent such as aloe vera.

While EXPANCEL® hollow microspheres as described above are preferred, it should be understood that other hollow microspheres having a density range of .005 g/cm³ to 1.24 g/cm³, preferably .05 g/cm³, could be used.

When prepared as described above, a preferred embodiment of the composite elastic layer will have a density of .5 g/cm³ to 1.3 g/cm³, preferably .94 g/cm³; a tensile strength greater than 1 Pa, preferably greater than .5 Pa; a durometer (00) of 13 to 62, preferably 22; a 100% modulus of 5 kPa to 250, preferably 20 kPa; and a compression set of 0 to 30, preferably 8.

It should be understood that different or additional skin treating agents may be utilized, depending upon the skin condition to be treated by the skin treating agent. For use as a typical suction sleeve, Vaseline and aloe vera are believed to provide good properties for the composite elastic layer that typically directly contacts or is in close proximity with the skin of a prosthetic user.

When the composite elastic material 12 is laminated or bonded with an elasticized textile layer 14, such layer 14, in a preferred embodiment, may be described as a Supplex Nylon circular knit of 87% Nylon, 13% Spandex fibers using 28 needles per 2.5 cm having a weight per square yard of 6.9 ozs. and a weight per linear yard of 12 ozs. Such a Supplex Nylon is obtainable from Agmont Inc. of Montreal, Quebec, Canada under Style Name 5095. This material has a finished width of 60" (152.4 cm) and is substantially elastically distendable along its length and width in a manner appropriate for a prosthetic suction liner.

The reinforcing fibers 26 may be a circular knit textile formed of relatively non-distendable fibers (at least within the load ranges contemplated for use in a prosthetic suction liner) wherein the knit construction is such that the layer 26 strongly resists elongation in a longitudinal direction while being freely distendable laterally in a radial direction when it is embedded in the composite elastic layer 12. Any appropriate reinforcement matrix that would provide such properties could be used for layer 26, but as a practical matter a circular knit glass fiber or Nylon material is appropriate, provided it has the anisotropic properties described above.

The textile layer 14 is normally air permeable and is usually formed from a flat knit elasticized fabric that has been rolled into a tube and stitched along abutting side edges along the length of the tube. The inside surface of the fabric layer 14 facing the composite elastic layer 12 is coated with a thin layer of cured silicone elastomer 24 that is partially embedded in the fibers of the textile 14 without completely penetrating the textile 14. The silicone elastomer layer 24 is cured while embedded in the textile so that it is firmly adhered to the textile and preferably renders the textile and silicone layer 24 impermeable to air. The thin coating of silicone elastomer 24 provides a good bonding surface for the composite elastic layer 12 described above.

Preferably, the silicone layer 24 is obtainable under Product No. CF 15-2188 from NuSil Technology of Carpinteria, California. Physical properties of the combined composite elastic layer 12, coating 24 and elasticized fabric 14 include a tensile strength greater than 1 Pa, preferably greater than 2 Pa; and a 100% modulus of 5 to 300 kPa, preferably 55 kPa.

The distal end cap 18 may be formed of a silicone elastomer including 98% by weight silicone rubber, type MED-4950 or type MED-4050 or type CF15-2188, all available from NuSil Technology, with the balance (2%) constituted of a color mixture, for example a color powder blended from 12.5 parts Lucas color No. 2408, 12.5 parts Lucas color No. 2439 and 75 parts Lucas color No. 2510 all obtainable from Fr. Schoenfeld GmbH and Co. Further properties of MED-4950 as published by NuSil Technology include: the material uses a platinum cure system; a press cure time of 50 minutes at 150°C; durometer 45 - 55; tensile strength 1000 psi (6.9 MPa); elongation 400%; and a tear strength 230 ppi (40.3 kN/M).

As illustrated in Figure 5, a sealing sleeve 30, for example a sleeve capable of sealing the gap between the upper end of a prosthetic socket and a residual limb as illustrated in Patent No. 5,571,208 includes an outer textile layer 32 that is an elasticized, porous or air permeable fabric on which a continuous cured silicone coating 34 has been applied and bonded thereto in the same manner as the coating 24 attached to the layer 14 of the suction sleeve material as illustrated in Figures 1 - 4 and described above.

The interior surface of the sleeve 30 includes a composite elastic material 36 formed in the same manner as the composite elastic layer 12 illustrated in Figures 1 - 4 and described above. The thickness of the composite elastic material 36 may be adjusted to fit the requirements of a sealing sleeve. The composite elastic layer 36 is intimately bonded and adhered to the coating 34. The combined assembly of the textile 32, coating 34 and composite elastic layer 36 is fully distendable both radially and longitudinally in accordance with the requirements of a sealing sleeve for prosthetic applications.

The outer fabric layer 32, in a preferred embodiment, may be a circular rib knit fabric made of 95% Nylon and 5% Lycra, knit as a 1X1 rib using 220 needles per 2.5 cm for a 12 cm width tube and 264 needles per 2.5 cm for a 14 cm tube. This fabric may be obtained from RX-Textile of Monroe, North Carolina.

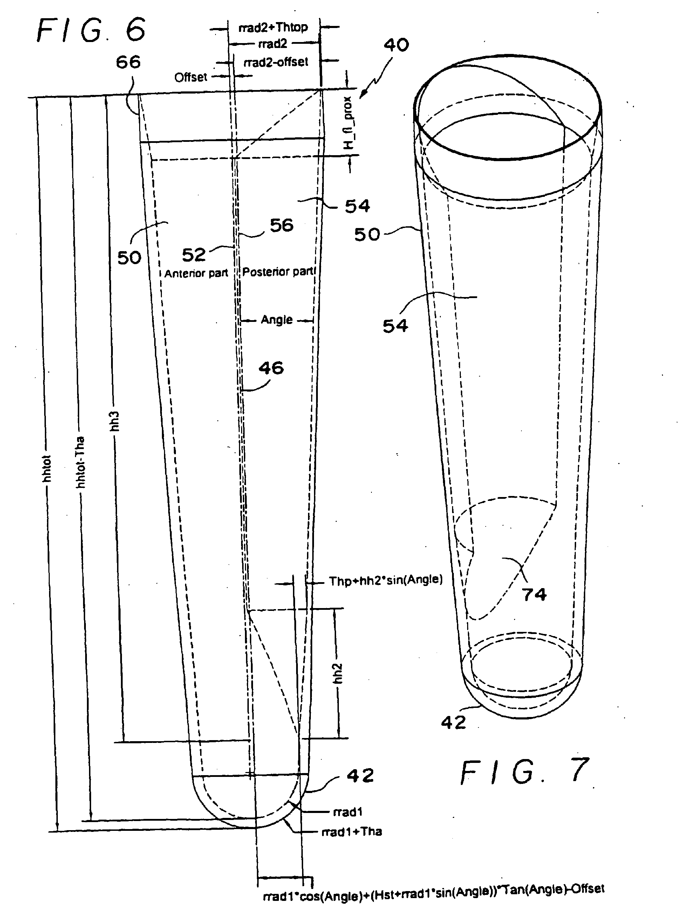

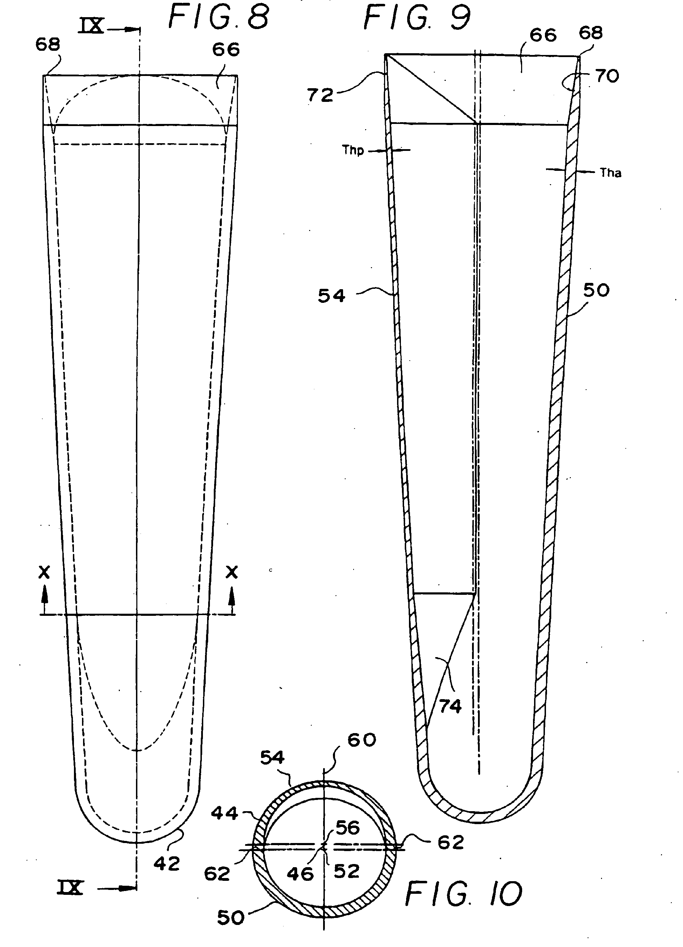

A preferred formed of the suction liner made with the composite elastic material layer 12 is illustrated in Figures 6 - 10 (the fabric is omitted in the veiws as being nonessential). The composite elastic material including the cured silicone elastomer layer with silicone oil and hollow microspheres and outer fabric is molded or formed as a tapered suction liner 40 having a closed distal end 42 of uniform thickness, an external profile 44 (see Figure 10) that is circular with the radii of curvature of the external surface 44 centered on a first central longitudinal axis 46 extending through the suction liner 40. The geometry of such suction liner is illustrated in Figures 6 - 10. Moreover, the following table 1 describes the variables shown in Figures 6 - 10 and also describes typical values of some of the variables for different size suction liners listed in the left column of the table entitled "Typical Values of Variables".

**TABLE 1**

| **VARIABLE DESCRIPTION** | |
|---|---|
| **Variable name** | **Description** |
| Angle | Angle of socket opening |
| H_fl_prox | Height of flange in proximal area |
| HH1 | Height to flange in distal area |
| HH2 | Height of flange in distal area |
| HH3 | Height of second cut |
| HHtot | Total height of socket |
| Hst | Height from radius to start of distal flange |
| Offset | Offset in lathe |
| Rrad1 | Radius on Distal end |
| RRad2 | Radius on proximal end |
| Tha | Thickness in anterior area |
| Thp | Thickness in posterior area |
| Thtop | Thickness of socket in top |

### TYPICAL VALUES OF VARIABLES

| **Size** | **Rrad1** | **HH1** | **HH2** | **HH3** | **Hhtot** | **RRad2** | **Tha** | **Angle** | **Hfh prox** | **Offset** | **Thp** | **Hst** | **Thtop** |
|---|---|---|---|---|---|---|---|---|---|---|---|---|---|
| 12 | 19 | rrad1+Hst | 80 | Hhtot-HH1-Tha | 450 | 44.1 | 6 | 3 | 40 | 3 | 3 | 20 | 1 |
| 14 | 22.5 | rrad1+Hst | 80 | Hhtot-HH1-Tha | 450 | 48.1 | 6 | 3 | 40 | 3 | 3 | 20 | 1 |
| 16 | 25.5 | rrad1+Hst | 80 | Hhtot-HH1-Tha | 450 | 51 | 6 | 3 | 40 | 3 | 3 | 20 | 1 |
| 18 | 28.5 | rrad1+Hst | 80 | Hhtot-HH1-Tha | 450 | 53.8 | 6 | 3 | 40 | 3 | 3 | 20 | 1 |
| 20 | 31.5 | rrad1+Hst | 80 | Hhtot-HH1-Tha | 450 | 56.7 | 6 | 3 | 40 | 3 | 3 | 20 | 1 |
| 21 | 33.5 | rrad1+Hst | 80 | Hhtot-HH1-Tha | 450 | 58.6 | 6 | 3 | 40 | 3 | 3 | 20 | 1 |
| 22 | 35.5 | rrad1+Hst | 80 | Hhtot-HH1-Tha | 450 | 60.5 | 6 | 3 | 40 | 3 | 3 | 20 | 1 |
| 23.5 | 37.5 | rrad1+Hst | 80 | Hhtot-HH1-Tha | 450 | 62.4 | 6 | 3 | 40 | 3 | 3 | 20 | 1 |
| 25 | 40 | rrad1+Hst | 80 | Hhtot-HH1-Tha | 450 | 64.7 | 6 | 3 | 40 | 3 | 3 | 20 | 1 |
| 26.5 | 42.5 | rrad1+Hst | 80 | Hhtot-HH1-Tha | 450 | 67.1 | 6 | 3 | 40 | 3 | 3 | 20 | 1 |
| 28 | 45 | rrad1+Hst | 80 | Hhtot-HH1-Tha | 450 | 69.5 | 6 | 3 | 40 | 3 | 3 | 20 | 1 |
| 30 | 48 | rrad1+Hst | 80 | Hhtot-HH1-Tha | 450 | 72.3 | 6 | 3 | 40 | 3 | 3 | 20 | 1 |
| 32 | 51 | rrad1+Hst | 80 | Hhtot-HH1-Tha | 450 | 75.1 | 6 | 3 | 40 | 3 | 3 | 20 | 1 |
| 34 | 54 | rrad1+Hst | 80 | Hhtot-HH 1-Tha | 450 | 78 | 6 | 3 | 40 | 3 | 3 | 20 | 1 |
| 36 | 57 | rrad1+Hst | 80 | Hhtot-HH1-Tha | 450 | 80.8 | 6 | 3 | 40 | 3 | 3 | 20 | 1 |
| 38 | 60.5 | rrad1+Hst | 80 | Hhtot-HH1-Tha | 450 | 84.2 | 6 | 3 | 40 | 3 | 3 | 20 | 1 |
| 40 | 64 | rrad1+Hst | 80 | Hhtot-HH1-Tha | 450 | 87.5 | 6 | 3 | 40 | 3 | 3 | 20 | 1 |
| 42.5 | 67.5 | rrad1+Hst | 80 | Hhtot-HH1-Tha | 450 | 90.8 | 6 | 3 | 40 | 3 | 3 | 20 | 1 |
| 45 | 71.5 | rrad1+Hst | 80 | Hhtot-HH1-Tha | 450 | 94.6 | 6 | 3 | 40 | 3 | 3 | 20 | 1 |

The suction sleeve 40 includes a circular curved inside anterior wall portion 50 having first radii of curvature centered on a second longitudinal axis of anterior curvature 52 extending longitudinally through the suction sleeve towards the anterior side of the first central axis 46 and a posterior wall portion 54 having second radii of curvature centered on a third longitudinal axis 56 located in the posterior direction relative to the central axis 46, said first, second and third longitudinal axes 46,52 and 56 all lying in a common longitudinally and transversely extending imaginary plane 60 (Figure 10) bisecting the anterior and posterior wall portions 50,54 and wherein the second and third axes 52 and 56 are spaced apart a predetermined offset distance from each other on opposed sides of the first axis 46. Thus, this arrangement produces a posterior wall that is thinner than the anterior wall as shown in Figure 10.

The anterior and posterior wall portions 50,54 intersect each other along inner diametrically opposed transition wall portions 62 that extend tangentially relative to the adjoining anterior and posterior wall portions along the sleeve length, so that the interior wall surface of the suction liner along the transition areas 62 are free of rapid changes in thickness, curvature or cross-section profile, as seen best in Figure 10.

In the example illustrated, the radii of curvature of the inside surfaces of the anterior and posterior portions of the sleeve are equal to each other along their respective second and third axes, as observable in Figure 10. A formula for generating the interior profile of the suction sleeve shown in Figures 6 - 10 is indicated at the bottom of Figure 6, and such formula is used to control a computer assisted machine tool (e.g., lathe) used to form a male mold element that shapes the inner profile of the liner.

At the proximal area of the suction sleeve 40 (the open end of the sleeve) a flange area 66 is provided wherein the thickness of the composite elastic material progressively thins as the top edge 68 is approached. The inside surface of the flange portion 66 of the anterior wall 50, as seen in Figure 9, tapers inwardly as the top edge 68 is approached as shown at 70 and the outer surface of the proximal end of the flange portion 66 of posterior wall 54 also tapers inwardly as shown at 72 in Figure 9. Preferably, the top edge 68 of the sleeve is relatively thin as compared with the thickness of the remainder of the sleeve.

The distal end 42 of the sleeve is spherical in curvature and joins the adjoining side wall of the sleeve along a tangent so as to provide a smooth interior and exterior contour as the sleeve transitions between the tapered conical upper portion and the spherically curved closed end portion 42. The thickness of the end portion 42 may be the same thickness as the anterior wall 50. The posterior wall 54 transitions from the same thickness as the anterior wall at the distal end of the sleeve to a thinner wall section over the length of the sleeve in which the thinner wall section is desired. A smooth transition area 74 is provided between the thinner posterior wall section 54 and the full thickness of the wall portion of the sleeve at the distal end of the sleeve.

The invention is not limited to the specific embodiments and examples described herein, but rather extends to the full scope of the claims.

## Claims

1. A composite elastic material (12) for prosthetic applications, comprising:
at least one cured silicone elastomer layer (12) containing silicone oil,
wherein said composite elastic material (12) is in the form of a tubular prosthetic suction liner (10; 40) having a closed distal end (16), and dimensioned and configured so as to be rollable onto a distal end of a residual limb of a prosthetic device user,
**characterized in that** hollow microspheres (28) are dispersed throughout the elastomer layer (12).

2. A composite elastic material (12) for prosthetic applications, comprising:
at least one cured silicone elastomer layer (12) containing silicone oil,
an elasticized fabric layer (14) intimately bonded to one side of the silicone elastomer layer (12),
**characterized in that** said composite elastic material (12) is in the form of a tubular sleeve open at opposed ends with the silicone elastomer layer (12) covering the inside wall of the sleeve and the elasticized fabric (14) covering the exterior of the sleeve, wherein hollow microspheres (28) are dispersed throughout the elastomer layer (12).

3. The composite elastic material (12) as claimed in claim 1, including an elasticized fabric layer (14) intimately bonded to one side of the silicone elastomer layer (12).

4. The composite elastic material (12) as claimed in claim 3, including a distension controlling reinforcement matrix (26) embedded in the silicone elastomer layer (12) over a distal end area (16) of the suction liner (10; 40), said matrix (26) containing reinforcement elements that provide substantial stiffness against elongation of the liner (10; 40) in a direction along the liner length and which do not provide substantial resistance against distension of the silicone elastomer layer (12) in directions transverse to the liner length. '

5. The composite elastic material (12) as claimed in claim 4, wherein said reinforcement matrix (26) is a knit textile having anisotropic distension properties along orthogonal directions, such that the textile is relatively inextensible in a first direction and is relatively freely extensible in an orthogonal direction.

6. The composite elastic material (12) as claimed in any of claims 3 to 5 including a rigid prosthetic connector (20) element attached to the distal end (16) of said liner (10), said connector element (20) arranged to engage and retain a prosthetic pin connector usable by a prosthetic user, said connector element (20) embedded in a cured silicone elastomer distal end cap (18) adhered to the distal end (16) of said suction liner (10) and providing access to a prosthetic pin connector.

7. The composite elastic material (12) as claimed in any of claims 3 to 6, wherein said suction liner (10; 40) is tapered inwardly towards its distal end (16; 42), and wherein the liner (10; 40) has a circular outer wall having radii of curvature centered along a liner first longitudinal axis (46) of external symmetry extending longitudinally centrally within the liner (10; 40); a circular curved inside anterior wall portion (50) extending along a liner length, and having first radii of curvature centered on a second longitudinal axis (52) of anterior curvature extending longitudinally along said liner length and a circular curved inside posterior wall portion (54) having second radii of curvature centered on a third longitudinal axis (56) of posterior curvature extending along said liner length, said first, second and third longitudinal axes (46, 52, 56) lying in a common longitudinally and transversely extending plane (60) bisecting the anterior and posterior wall portions (50, 54), and wherein said second and third axes (52, 56) are spaced apart a predetermined offset distance on opposed sides of said first axis (46) to thereby define an anterior wall portion (50) that is thicker along said liner length than the posterior portion (54); and further wherein said anterior and posterior wall portions (50, 54) intersect each other along said liner length on the liner interior along diametrically opposed inner transition wall portions (62) that extend tangentially relative to the adjoining anterior and posterior wall portions (50, 54) along said liner length, whereby the interior wall of the suction liner (10; 40) along the inner transition wall portions (62) are free of rapid changes in thickness, curvature or cross-section profile.

8. The composite elastic material (12) as claimed in claim 7, wherein said second and third radii are equal to each other along their respective second and third axes (52, 56).

9. The composite elastic material (12) as claimed in claim 7 or 8, wherein an interior proximal flange area (66) of said anterior wall portion is tapered radially outwardly and an exterior proximal flange area of said posterior wall portion (54) is tapered radially inwardly.

10. The composite elastic material (12) as claimed in claims 7, 8 or 9, including a spherical curved inside distal wall portion (42) of said suction liner (40), said distal wall portion (42) joining the adjoining interior wall of the suction liner (40) along a tangency that forms a smooth transition (74) between the inside distal wall portion and the adjoining interior wall of the suction liner (40), and further wherein the adjoining wall of the suction liner (40) is circular in cross-section and has a center of curvature located on said first axis (46); and further wherein the thickness of the wall of the suction liner (40) adjoining said spherical curved distal wall portion (42) is uniform and equal to the thickness of said anterior wall portion (50).

11. The composite elastic material (12) as claimed in any of claims 2 - 10, including a second cured silicone elastomer layer (24) between the at least one silicone elastomer layer (12) and the elasticized fabric layer (14), said second silicone elastomer layer (24) partially penetrating and being embedded in said textile layer (14) on one side thereof so as to form a continuous coating on said one side, and adhered to said at least one silicone elastomer layer (12) on the opposite side of the second silicone elastomer layer (24).

12. The composite elastic material (12) as claimed in claim 2 or 11, wherein said elasticized fabric layer (14) is a normally porous, air permeable material that has been rendered non-air permeable by a second silicone cured elastomer layer (24), said second silicone elastomer layer (24) forming a relatively thin coating as compared with the elasticized fabric layer (14) on said one side of said fabric layer (14).

13. The composite elastic material (12) as claimed in claim 2 or 12, wherein said elasticized fabric layer (14) comprises a rectangular section of flat elasticized fabric that is folded into a tube and is sewn together along one side of the tube along abutting longitudinally extending edges of the fabric.

14. The composite elastic material (12) as claimed in any of claims 3 to 13, wherein the composite elastic material (12) has a minimum tensile strength of one Pa and a 100% modulus of 5 to 30 kPa.

15. The composite elastic material (12) as claimed in claim 14, wherein the elasticized fabric (14) is supplex Nylon jersey knit, 28 needles per 2.5 cm, comprising 87% Nylon, 13% Spandex, said fabric (14) substantially stretchable beyond its relaxed dimensions both lengthwise and widthwise.

16. The composite elastic material (12) as claimed in claim 2, wherein the elasticized fabric (14) is circular rib knit of 95% Nylon and 5% Lycra, 220 needles per 2.5 cm in 12 cm width and 264 needles per 2.5 cm in 14 cm width, from Rx Textile.

17. The composite elastic material (12) as claimed in any of the above claims, wherein said microspheres (28) are expanded polymeric shells.

18. The composite elastic material (12) as claimed in any of the above claims, wherein said microspheres (28) have a density of .005 g/cm³ to 1.25 g/cm³.

19. The composite elastic material (12) as claimed in claim 18, wherein said microspheres (28) have a density of .05 g/cm³.

20. The composite elastic material (12) as claimed in any of the above claims, wherein said at least one silicone elastomer layer (12) comprises, by weight:
50 - 99.4% silicone elastomer
.5 - 45% silicone oil
.1 - 5% microspheres (28).

21. The composite elastic material (12) as claimed in claim 20, wherein the ratios of silicone elastomer, silicone oil and microspheres (28) are as follows, by weight:
77.25% silicone elastomer
10% silicone oil
.75% microspheres (28).

22. The composite elastic material (12) as claimed in any of claims 1 - 20, including one or more skin treatment agents blended with the silicone elastomer.

23. The composite elastic material (12) as claimed in claim 22, wherein the skin treatment agent consists of Vaseline, said Vaseline present in an amount of up to 15% by weight of the silicone elastomer layer (12).

24. The composite elastic material (12) as claimed in claim 22 wherein the skin treatment agent comprises Vaseline and aloe vera.

25. The composite elastic material (12) as claimed in claim 24, wherein the aloe vera constitutes 3% by weight of the at least one silicone elastomer layer (12) and the balance of the skin treatment agents is Vaseline, said skin treatment agents constituting up to 20% by weight of the silicone elastomer layer (12).

26. The composite elastic material (12) as claimed in any of the above claims, wherein the at least one silicone elastomer layer (12) has the following properties:
| | |
|---|---|
| Density : | .5g/cm³ to 1.3 g/cm³ |
| Tensile Strength : | .1 Pa minimum |
| Durometer (00) : | 13 - 62 |
| 100% Modulus : | 5 kPa to 250 kPa |
| Compression Set : | 0 to 30 |

27. The composite elastic material (12) as claimed in claim 26, wherein the silicone elastomer layer (12) has the following properties:
| | |
|---|---|
| Density : | .94 g/cm³ |
| Tensile Strength : | .5 Pa |
| Durometer (00) : | 22 |
| 100% Modulus : | 20 kPa |
| Compression Set : | 8 |

28. The composite elastic material (12) as claimed in claim 3, wherein the at least one silicone elastomer layer (12) comprises by weight:
77.25% CF13-2188 NuSil Technology Silicone Elastomer;
10% Baysilone Fluid M350 silicone oil of GE Bayer Silicones GmbH;
11.9% Medical grade type WebCo 71 Vaseline of Shell;
.75% Expancel expanded 551 DE microspheres of AKZO NOBEL; and
.1% Aloe Vera Pure Beauty oil of Jason Natural Cosmetics, product 78522-04001.

29. The composite elastic material (12) as claimed in claim 3, wherein the at least one silicone elastomer layer (12) comprises by weight:
77.25% CF13-2188 NuSil Technology Silicone Elastomer;
10% Baysilone Fluid M350 silicone oil of GE Bayer Silicones GmbH;
11.9% Medical grade type WebCo 71 Vaseline of Shell;
.75% Expancel expanded 551 DE microspheres of AKZO NOBEL;
.1% Aloe Vera Pure Beauty oil of Jason Natural Cosmetics, product 78522-04001;
and further including a cured silicone elastomer distal end cap (18) adhered to the distal outer end of said suction liner (10; 40), said end cap (18) formed of MED-4950 or MED-4050 or CF 15-2188 of NuSil Technology and 2% by weight of coloring powder.

## Patentansprüche

1. Elastischer Verbundwerkstoff (12) für prothetische Anwendungen, umfassend:
mindestens eine Silikonöl enthaltende, gehärtete Silikonelastomerschicht (12),
wobei der elastische Verbundwerkstoff (12) die Form eines rohrförmigen prothetischen Saug-Liners (10; 40) mit einem geschlossenen Distalende (16) aufweist und so dimensioniert und aufgebaut ist, dass er auf das Distalende einer Restgliedmaße eines Benutzers einer Prothesenvorrichtung aufrollbar ist,
**dadurch gekennzeichnet, dass** hohle Mikrokugeln (28) überall in der Elastomerschicht (12) verteilt sind.

2. Elastischer Verbundwerkstoff (12) für prothetische Anwendungen, umfassend:
mindestens eine Silikonöl enthaltende, gehärtete Silikonelastomerschicht (12),
eine mit einer Seite der Silikonelastomerschicht (12) innig verbundene, elastifizierte Stoffschicht (14),
**dadurch gekennzeichnet, dass** der elastische Verbundwerkstoff (12) die Form eines an gegenüberliegenden Enden offenen, rohrförmigen Schlauchs aufweist, wobei die Silikonelastomerschicht (12) die Innenwand des Schlauchs bedeckt und die elastifizierte Stoffschicht (14) das Äußere des Schlauchs bedeckt, wobei hohle Mikrokugeln (28) überall in der Elastomerschicht (12) verteilt sind.

3. Elastischer Verbundwerkstoff (12) nach Anspruch 1, umfassend eine mit einer Seite der Silikonelastomerschicht (12) innig verbundene, elastifizierte Stoffschicht (14).

4. Elastischer Verbundwerkstoff (12) nach Anspruch 3, umfassend eine Dehnung regulierende Verstärkungsmatrix (26), welche in der Silikonelastomerschicht (12) über einem Distalendbereich (16) des Saug-Liners (10; 40) eingebettet ist, wobei die Matrix (26) Verstärkungselemente enthält, die erhebliche Festigkeit gegen Ausdehnung des Liners (10; 40) in einer Richtung entlang der Länge des Liners bieten und die keinen erheblichen Widerstand gegen Dehnung der Silikonelastomerschicht (12) in Richtungen quer zur Länge des Liners bieten.

5. Elastischer Verbundwerkstoff (12) nach Anspruch 4, wobei die Verstärkungsmatrix (26) ein Textilgewirk ist, welches anisotrope Dehnungseigenschaften entlang orthogonaler Richtungen aufweist, so dass das Textil relativ undehnbar in einer ersten Richtung und relativ frei dehnbar in einer orthogonalen Richtung ist.

6. Elastischer Verbundwerkstoff (12) nach einem der Ansprüche 3 bis 5, umfassend ein an dem Distalende (16) des Liners (10) angebrachtes, festes Prothesenverbinderelement (20), wobei das Verbinderelement (20) eingerichtet ist, mit einem von einem Prothesenbenutzer benutzbaren Prothesenstiftverbinder zusammenzuwirken und diesen zu halten, wobei das Verbindungselement (20) in einer gehärteten Silikonelastomer-Distalendkappe (18) eingebettet ist, welche an dem Distalende (16) des Saug-Liners (10) anhaftet und Anschluss an einen Prothesenstiftverbinder bietet.

7. Elastischer Verbundwerkstoff (12) nach einem der Ansprüche 3 bis 6, wobei der Saug-Liner (10; 40) in Richtung seines Distalendes (16; 42) konisch zuläuft, und wobei der Liner (10; 40) eine kreisförmige Außenwand mit Krümmungsradien aufweist, die entlang einer sich längs mittig innerhalb des Liners (10; 40) erstreckenden ersten längslaufenden äußeren Symmetrieachse (46) zentriert sind; wobei sich ein kreisförmiger, gebogener, innerer, vorderer Wandbereich (50) entlang einer Länge des Liners erstreckt und erste Krümmungsradien aufweist, die auf einer sich längs entlang der Länge des Liners erstreckenden zweiten längslaufenden vorderen Krümmungsachse (52) zentriert sind, und ein kreisförmiger, gebogener, innerer, hinterer Wandbereich (54) mit zweiten Krümmungsradien, die auf einer sich entlang der Länge des Liners erstreckenden dritten längslaufenden hinteren Krümmungsachse (56) zentriert sind, wobei die erste, zweite und dritte Längsachse (46, 52, 56) in einer gemeinsamen sich längs und quer erstreckenden Ebene (60) liegen, welche den vorderen und hinteren Wandteil (50, 54) schneidet, und wobei die zweite und dritte Achse (52, 56) mit einem vorgegebenen Versatzabstand auf gegenüberliegenden Seiten der ersten Achse (46) voneinander beabstandet sind, um **dadurch** einen vorderen Wandbereich (50) zu definieren, welcher entlang der Länge des Liners dicker ist als der hintere Bereich (54); und des Weiteren wobei sich der vordere und hintere Wandbereich (50, 54) entlang der Länge des Liners im Innern des Liners entlang diametral entgegengesetzten inneren Übergangswandbereichen (62) schneiden, welche sich tangential relativ zu den aneinandergrenzenden vorderen und hinteren Wandbereichen (50, 54) entlang der Länge des Liners erstrecken, wobei die Innenwand des Saug-Liners (10; 40) entlang den inneren Übergangswandbereicheri (62) frei von schnellen Änderungen der Dicke, Krümmung oder des Querschnittprofils ist.

8. Elastischer Verbundwerkstoff (12) nach Anspruch 7, wobei der zweite und dritte Radius entlang ihrer entsprechenden zweiten und dritten Achse (52, 56) gleich sind.

9. Elastischer Verbundwerkstoff (12) nach Anspruch 7 oder 8, wobei ein innerer, proximaler Flanschbereich (66) des vorderen Wandbereichs radial auswärts angeschrägt ist und ein äußerer proximaler Flanschbereich des hinteren Wandbereichs (54) radial einwärts angeschrägt ist.

10. Elastischer Verbundwerkstoff (12) nach Anspruch 7, 8 oder 9, umfassend einen sphärischen, gekrümmten, inneren, distalen Wandbereich (42) des Saug-Liners (40), wobei der distale Wandbereich (42) mit der angrenzenden inneren Wand des Saug-Liners (40) entlang einer Berührung, welche einen glatten Übergang (74) zwischen dem inneren distalen Wandbereich und der angrenzenden inneren Wand des Saug-Liners (40) bildet, verbunden ist, und des Weiteren wobei die angrenzende Wand des Saug-Liners (40) einen kreisförmigen Querschnitt hat und ein Krümmungszentrum aufweist, welches sich auf der ersten Achse (46) befindet; und des Weiteren wobei die Dicke der Wand des Saug-Liners (40), welche an den sphärischen, gekrümmten, distalen Wandbereich (42) angrenzt, gleichförmig und gleich der Dicke des vorderen Wandbereichs (50) ist.

11. Elastischer Verbundwerkstoff (12) nach einem der Ansprüche 2 bis 10, umfassend eine zweite gehärtete Silikonelastomerschicht (24) zwischen der zumindest einen Silkonelastomerschicht (12) und der elastifizierten Stoffschicht (14), wobei die zweite Silkonelastomerschicht (24) auf einer Seite der Stoffschicht (14) diese teilweise durchdringt und in diese eingebettet ist, um eine zusammenhängende Beschichtung auf der einen Seite zu bilden, und an der zumindest einen Silkonelastomerschicht (12) auf der gegenüberliegenden Seite der zweiten Silikonelastomerschicht (24) anhaftet.

12. Elastischer Verbundwerkstoff (12) nach Anspruch 2 oder 11, wobei die elastifizierte Stoffschicht (14) ein normalerweise poröses, luftdurchlässiges Material ist, welches durch eine zweite gehärtete Silikonelastomerschicht (24) luftundurchlässig gemacht wurde, wobei die zweite Silikonelastomerschicht (24) eine relativ dünne Beschichtung verglichen mit der elastifizierten Stoffschicht (14) auf der einen Seite der Stoffschicht (14) bildet.

13. Elastischer Verbundwerkstoff (12) nach Anspruch 2 oder 12, wobei die elastifizierte Stoffschicht (14) einen rechteckigen Abschnitt flachen elastifizierten Stoffes umfasst, welcher zu einer Röhre gefaltet ist und entlang einer Seite der Röhre entlang aneinander stoßenden, sich längs erstreckenden Rändern des Stoffes zusammen genäht ist.

14. Elastischer Verbundwerkstoff (12) nach einem der Ansprüche 3 bis 13, wobei der elastische Verbundwerkstoff (12) eine minimale Zugfestigkeit von einem Pa und einen 100%-Modul von 5 bis 30 kPa hat.

15. Elastischer Verbundwerkstoff (12) nach Anspruch 14, wobei der elastifizierte Stoff (14) Supplex Nylon Jerseygewirk ist, 28 Nadeln pro 2,5 cm, umfassend 87% Nylon, 13% Spandex, wobei der Stoff (14) erheblich dehnbar über seine entspannten Abmessungen in Längen- und Breitenrichtung ist.

16. Elastischer Verbundwerkstoff (12) nach Anspruch 2, wobei der elastifizierte Stoff (14) kreisförmiges Rippengewirk von 95% Nylon und 5% Lycra ist, 220 Nadeln pro 2,5 cm bei 12 cm Breite und 264 Nadeln pro 2,5 cm bei 14 cm Breite, von Rx Textile.

17. Elastischer Verbundwerkstoff (12) nach einem der obigen Ansprüche, wobei die Mikrokugeln (28) geschäumte Polymerhülsen sind.

18. Elastischer Verbundwerkstoff (12) nach einem der obigen Ansprüche, wobei die Mikrokugeln (28) eine Dichte von 0,005 g/cm³ bis 1,25 g/cm³ haben.

19. Elastischer Verbundwerkstoff (12) nach Anspruch 18, wobei die Mikrokugeln (28) eine Dichte von 0,05 g/cm³ haben.

20. Elastischer Verbundwerkstoff (12) nach einem der obigen Ansprüche, wobei die zumindest eine Silikonelastomerschicht (12) umfasst, nach Gewicht:
50 - 99,4% Silikonelastomer
0,5 - 45% Silikonöl
0,1 - 5% Mikrokugeln (28).

21. Elastischer Verbundwerkstoff (12) nach Anspruch 20, wobei die Verhältnisse von Silikonelastomer, Silikonöl und Mikrokugeln (28) wie folgt sind, nach Gewicht:
77,25% Silikonelastomer
10% Silikonöl
0,75% Mikrokugeln (28).

22. Elastischer Verbundwerkstoff (12) nach einem der Ansprüche 1 bis 20, umfassend ein oder mehrere mit dem Silikonelastomer vermischte Hautbehandlungsmittel.

23. Elastischer Verbundwerkstoff (12) nach Anspruch 22, wobei das Hautbehandlungsmittel aus Vaseline besteht, wobei die Vaseline in einer Menge von bis zu 15 Gewichts-% der Silikonelastomerschicht (12) vorliegt.

24. Elastischer Verbundwerkstoff (12) nach Anspruch 22, wobei das Hautbehandlungsmittel Vaseline und Aloe Vera umfasst.

25. Elastischer Verbundwerkstoff (12) nach Anspruch 24, wobei das Aloe Vera 3 Gewichts-% der zumindest einen Silikonelastomerschicht (12) ausmacht und der Rest der Hautbehandlungsmittel Vaseline ist, wobei die Hautbehandlungsmittel bis zu 20 Gewichts-% der Silikonelastomerschicht (12) ausmachen.

26. Elastischer Verbundwerkstoff (12) nach einem der obigen Ansprüche, wobei die zumindest eine Silikonelastomerschicht (12) die folgenden Eigenschaften hat:
| | |
|---|---|
| Dichte : | 0,5 g/cm³ bis 1,3 g/cm³ |
| Zugfestigkeit : | mindestens 0,1 Pa |
| Durometer (00) : | 13 - 62 |
| 100%-Modul : | 5 kPa bis 250 kPa |
| Druckverformung : | 0 bis 30 |

27. Elastischer Verbundwerkstoff (12) nach Anspruch 26, wobei die Silikonelastomerschicht (12) die folgenden Eigenschaften hat:
| | |
|---|---|
| Dichte : | 0,94 g/cm³ |
| Zugfestigkeit : | 0,5 Pa |
| Durometer (00) : | 22 |
| 100%-Modul : | 20 kPa |
| Druckverformung : | 8 |

28. Elastischer Verbundwerkstoff (12) nach Anspruch 3, wobei die zumindest eine Silikonelastomerschicht (12) nach Gewicht umfasst:
77,25% CF13-2188 NuSil Technology Silikonelastomer;
10% Baysilone Fluid M350 Silikonöl von GE Bayer Silicones GmbH;
11,9% Medical grade Typ WebCo 71 Vaseline von Shell;
0,75% Expancel geschäumte 551 DE Mikrokugeln von AKZO NOBEL; und
0,1% Aloe Vera Pure Beauty Oil von Jason Natural Cosmetics, Produkt 78522-04001.

29. Elastischer Verbundwerkstoff (12) nach Anspruch 3, wobei die zumindest eine Silikonelastomerschicht (12) nach Gewicht umfasst:
77,25% CF13-2188 NuSil Technology Silikonelastomer;
10% Baysilone Fluid M350 Silikonöl von GE Bayer Silicones GmbH;
11,9% Medical grade Typ WebCo 71 Vaseline von Shell;
0,75% Expancel geschäumte 551 DE Mikrokugeln von AKZO NOBEL; und
0,1% Aloe Vera Pure Beauty Oil von Jason Natural Cosmetics, Produkt 78522-04001;
und des Weiteren umfassend eine gehärtete Silikonelastomer-Distalendkappe (18), welche mit dem distalen äußeren Ende des Saug-Liners (10; 40) verbunden ist, wobei die Endkappe (18) aus MED-4950 oder MED-4050 oder CF 15-2188 von NuSil Technology und 2 Gewichts-% Farbpulver gebildet ist.

## Revendications

1. Matériau élastique composite (12) destiné à des applications prothétiques, comprenant :
au moins une couche élastomère de silicone durcie (12) contenant de l'huile de silicone, dans lequel ledit matériau élastique composite (12) se présente sous la forme d'une gaine à succion prothétique tubulaire (10 ; 40) comprenant une extrémité distale fermée (16), dimensionnée et configurée de manière à pouvoir être roulée sur une extrémité distale d'un membre résiduel d'un utilisateur de dispositif prothétique,
**caractérisé en ce que** des microsphères creuses (28) sont dispersées dans l'ensemble de la couche élastomère (12).

2. Matériau élastique composite (12) destiné à des applications prothétiques, comprenant :
au moins une couche élastomère de silicone durcie (12) contenant de l'huile de silicone,
une couche de tissu élastique (14) intimement liée sur un côté de la couche élastomère de silicone (12),
**caractérisé en ce que** ledit matériau élastique composite (12) se présente sous la forme d'un manchon tubulaire ouvert au niveau des extrémités opposées, la couche élastomère de silicone (12) couvrant la paroi intérieure du manchon et le tissu élastique (14) couvrant l'extérieur du manchon, dans lequel des microsphères creuses (28) sont dispersées dans l'ensemble de la couche élastomère (12).

3. Matériau élastique composite (12) selon la revendication 1, incluant une couche de tissu élastique (14) intimement liée sur un côté de la couche élastomère de silicone (12).

4. Matériau élastique composite (12) selon la revendication 3, incluant une matrice de renforcement régulant la distension (26) noyée dans la couche élastomère de silicone (12) sur une zone d'extrémité distale (16) de la gaine à succion (10 ; 40), ladite matrice (26) contenant des éléments de renforcement qui confèrent une rigidité significative contre l'allongement de la gaine (10 ; 40) dans une direction suivant la longueur de la gaine, mais qui n'opposent pas de résistance significative à la distension de la couche élastomère de silicone (12) dans les directions transversales à la longueur de gaine.

5. Matériau élastique composite (12) selon la revendication 4, dans lequel ladite matrice de renforcement (26) est un textile tricoté présentant des propriétés de distension anisotropes le long des directions orthogonales, de sorte que le textile est relativement non extensible dans une première direction et est extensible de manière relativement libre dans une direction orthogonale.

6. Matériau élastique composite (12) selon l'une quelconque des revendications 3 à 5, incluant un élément connecteur prothétique rigide (20), fixé à l'extrémité distale (16) de ladite gaine (10), ledit élément connecteur (20) étant agencé de manière à venir en prise avec un connecteur à broche prothétique pouvant être utilisé par un utilisateur de prothèse, et à retenir celui-ci, ledit élément connecteur (20) étant noyé dans un bouchon d'extrémité distale élastomère de silicone durcie (18) collé sur l'extrémité distale (18) de ladite gaine à succion (10) et donnant accès à un connecteur de broche prothétique.

7. Matériau élastique composite (12) selon l'une quelconque des revendications 3 à 6, dans lequel ladite gaine à succion (10 ; 40) diminue progressivement vers l'intérieur en direction de son extrémité distale (16; 42), et dans lequel la gaine (10 ; 40) présente une paroi extérieure circulaire présentant des rayons de courbure centrés le long d'un premier axe longitudinal de gaine (46) de symétrie externe s'étendant de manière longitudinale et centrale à l'intérieur de la gaine (10 ; 40) ; une partie de paroi antérieure intérieure incurvée et circulaire (50) s'étendant le long d'une longueur de gaine, et présentant des premiers rayons de courbure centrés sur un deuxième axe longitudinal (52) de courbure antérieure s'étendant de manière longitudinale le long de ladite longueur de gaine, et une partie de paroi postérieure intérieure incurvée et circulaire (54) présentant des seconds rayons de courbure centrés sur un troisième axe longitudinal (56) de courbure postérieure s'étendant le long de ladite longueur de gaine, lesdits premier, deuxième et troisième axes (46, 52, 46) se trouvant dans un plan commun s'étendant de manière longitudinale et transversale (60) coupant les parties de paroi antérieure et postérieure (50, 54), et dans lequel lesdits deuxième et troisième axes (52, 56) sont espacés l'un de l'autre d'une distance de décalage prédéterminée sur les côtés opposés dudit premier axe (46), afin de définir ainsi une partie de paroi antérieure (50) plus épaisse le long de ladite longueur de gaine que la partie postérieure (54), et dans lequel en outre, lesdites parties de paroi antérieure et postérieure (50, 54) se croisent l'une avec l'autre le long de ladite longueur de gaine sur l'intérieur de gaine le long de parties de paroi de transition intérieures diamétralement opposées (62) qui s'étendent de manière tangentielle par rapport aux parties de paroi antérieure et postérieure attenantes (50, 54) le long de ladite longueur de gaine, la paroi intérieure de la gaine à succion (10 ; 40) le long des parties de paroi de transition intérieure (62) étant exempte de changements rapides d'épaisseur, de courbure ou de profil en travers.

8. Matériau élastique composite (12) selon la revendication 7, dans lequel lesdits deuxièmes et troisièmes rayons sont égaux entre eux le long de leurs deuxième et troisième axes respectifs (52, 56).

9. Matériau élastique composite (12) selon la revendication 7 ou 8, dans lequel une zone de bride proximale intérieure (66) de ladite partie de paroi antérieure diminue progressivement de manière radiale vers l'extérieur, et une zone de bride proximale extérieure de ladite partie de paroi postérieure (54) diminue progressivement de manière radiale vers l'intérieur.

10. Matériau élastique composite (12) selon la revendication 7, 8 ou 9, incluant une partie de paroi distale intérieure incurvée et sphérique (42) de ladite gaine à succion (40), ladite partie de paroi distale (42) joignant la paroi intérieure attenante de la gaine à succion (40) le long d'une tangence qui forme une transition lisse (74) entre la partie de paroi distale intérieure et la paroi intérieure attenante de la gaine à succion (40), dans lequel en outre la paroi attenante de la gaine à succion (40) est de section circulaire et présente un centre de courbure se situant sur ledit premier axe (46), et dans lequel, en outre, l'épaisseur de la paroi de la gaine à succion (40) attenante à ladite partie de paroi distale incurvée sphérique (42) est uniforme et égale à l'épaisseur de ladite partie de paroi antérieure (50).

11. Matériau élastique composite (12) selon l'une quelconque des revendications 2 à 10, incluant une seconde couche élastomère de silicone durcie (24) entre la au moins une couche élastomère de silicone (12) et la couche de tissu élastique (14), ladite seconde couche élastomère de silicone (24) entrant partiellement et étant noyée dans ladite couche textile (14) sur un des côtés de celle-ci de manière à former un revêtement continu sur ledit un côté, et étant collée à ladite au moins une couche élastomère de silicone (12) sur le côté opposé de la seconde couche élastomère de silicone (24).

12. Matériau élastique composite (12) selon la revendication 2 ou 11, dans lequel ladite couche de tissu élastique (14) est un matériau normalement poreux et perméable à l'air ayant été rendu imperméable à l'air par une seconde couche élastomère de silicone durcie (24), ladite seconde couche élastomère de silicone (24) formant un revêtement relativement mince par comparaison à la couche de tissu élastique (14) sur ledit un côté de ladite couche de tissu (14).

13. Matériau élastique composite (12) selon la revendication 2 ou 12, dans lequel ladite couche de tissu élastique (14) comprend une section rectangulaire de tissu élastique plat pliée en un tube et cousue ensemble le long d'un côté du tube le long de bords d'aboutement s'étendant de manière longitudinale du tissu.

14. Matériau élastique composite (12) selon l'une quelconque des revendications 3 à 13, dans lequel le matériau élastique composite (12) présente une résistance à la traction minimale de 1 Pa et un module à 100% de 5 à 30 kPa.

15. Matériau élastique composite (12) selon la revendication 14, dans lequel le tissu élastique (14) est un tricot de jersey en nylon supplex, 28 aiguilles par 2,5 cm, comprenant 87% de nylon et 13% d'élasthanne/spandex, ledit tissu (14) pouvant être en grande partie étiré au-delà de ses dimensions relaxées tant dans le sens de la longueur que de la largeur.

16. Matériau élastique composite (12) selon la revendication 2, dans lequel le tissu élastique (14) est un tricot à côtes circulaire avec 95% de nylon et 5% élasthanne/Lycra®, 220 aiguilles par 2,5 cm pour une largeur de 12 cm, et 264 aiguilles par 2,5 cm pour une largeur de 14 cm, disponible auprès de Rx Textile.

17. Matériau élastique composite (12) selon l'une quelconque des revendications précédentes, dans lequel lesdites microsphères (28) sont des enveloppes polymères expansées.

18. Matériau élastique composite (12) selon l'une quelconque des revendications précédentes, dans lequel lesdites microsphères (28) présentent une masse volumique de 0,005 g/cm³ à 1,25 g/cm³.

19. Matériau élastique composite (12) selon la revendication 18, dans lequel lesdites microsphères (28) présentent une masse volumique de 0,05 g/cm³.

20. Matériau élastique composite (12) selon l'une quelconque des revendications précédentes, dans lequel ladite au moins une couche élastomère de silicone (12) comprend, en poids :
50-99,4% d'élastomère de silicone ;
0,5-45% d'huile de silicone, et
0,1-5% de microsphères (28).

21. Matériau élastique composite (12) selon la revendication 20, dans lequel les rapports, en poids, entre l'élastomère de silicone, l'huile de silicone et les microsphères (28), sont les suivants :
77,25% d'élastomère de silicone ;
10% d'huile de silicone, et
0,75% de microsphères (28).

22. Matériau élastique composite (12) selon l'une quelconque des revendications 1 à 20, incluant un ou plusieurs agents de traitement de la peau mélangés à l'élastomère de silicone.

23. Matériau élastique composite (12) selon la revendication 22, dans lequel l'agent de traitement de la peau consiste en de la vaseline, ladite vaseline étant présente en une quantité allant jusqu'à 15% en poids de la couche élastomère de silicone (12).

24. Matériau élastique composite (12) selon la revendication 22, dans lequel l'agent de traitement de la peau comprend de la vaseline ou de l'aloès.

25. Matériau élastique composite (12) selon la revendication 24, dans lequel l'aloès constitue 3% en poids d'au moins une couche élastomère de silicone (12), et le reste des agents de traitement de la peau est de la vaseline, lesdits agents de traitement de la peau constituant jusqu'à 20% en poids de la couche élastomère de silicone (12).

26. Matériau élastique composite (12) selon l'une quelconque des revendications précédentes, dans lequel la au moins une couche élastomère de silicone (12) présente les propriétés suivantes :
| | |
|---|---|
| masse volumique : | 0,5 g/cm³ à 1,3 g/cm³ |
| résistance à la traction : | 0,1 Pa minimum |
| duromètre (00) : | 13-62 |
| Module 100% : | 5 kPa à 250 kPa |
| Déformation rémanente après compression : | 0 à 30. |

27. Matériau élastique composite (12) selon la revendication 26, dans lequel la couche élastomère de silicone (12) présente les propriétés suivantes :
| | |
|---|---|
| masse volumique : | 0,94 g/cm³ |
| résistance à la traction : | 0,5 Pa |
| duromètre (00) : | 22 |
| Module 100% : | 20 kPa |
| Déformation rémanente après compression : | 8. |

28. Matériau élastique composite (12) selon la revendication 3, dans lequel la au moins une couche élastomère de silicone (12) comprend en poids :
| | |
|---|---|
| 77,25% | d'élastomère CF13-2188 NuSil Technology Silicone Elastomer ; |
| 10% | d'huile de silicone Baysilone Fluid M350 de GE Bayer Silicones GmbH ; |
| 11,9% | de vaseline de qualité médicale type WebCo 71 de Shell ; |
| 0,75% | de microsphères expansées à l'Expancel 551 DE de AKZO NOBEL, et |
| 0,1% | d'huile d'aloès Aloe Vera Pure Beauty de Jason Natural Cosmetics, |
produit 78522-04001.

29. Matériau élastique composite (12) selon la revendication 3, dans lequel la au moins une couche élastomère de silicone (12) comprend en poids :
| | |
|---|---|
| 77,25% | d'élastomère CF13-2188 NuSil Technology Silicone Elastomer ; |
| 10% | d'huile de silicone Baysilone Fluid M350 de GE Bayer Silicones GmbH ; |
| 11,9% | de vaseline de qualité médicale type WebCo 71 de Shell ; |
| 0,75% | de microsphères expansées à l'Expancel 551 DE de AKZO NOBEL, et |
| 0,1% | d'huile d' aloès Aloe Vera Pure Beauty de Jason Natural Cosmetics, produit 78522-04001, et |
incluant en outre un bouchon d'extrémité distale élastomère de silicone durcie (18) collé à l'extrémité extérieure distale de ladite gaine à succion (10 ; 40), ledit bouchon d'extrémité (18) formé de MED-4950, de MED-4050, ou de CF 15-2188 de NuSil Technology, et de 2% en poids de poudre colorante.
